# FASCICULE DE BREVET EUROPEEN

(11) **EP 0 563 346 B1**
(45) Date de publication et mention de la délivrance du brevet: **26.02.1997**
(21) Numéro de dépôt: 92920332.1
(22) Date de dépôt: 06.10.1992
(51) Int. Cl.: C12P 7/40, C12P 11/00, C12P 17/04

(54) **Procédé de production d'acides carboxyliques à l'aide de microoganismes**
Verfahren zur Herstellung von Karbonsäuren mit Hilfe von Mikroorganismen
Method for the production of carboxilic acid using microrganisms

(30) Priorité: 18.10.1991 CH 3056/91
(43) Date de publication de la demande: 06.10.1993
(73) Titulaire: FIRMENICH SA, CH-1211 Genève 8 (CH)
(72) Inventeur: WHITEHEAD, Ian, Michael, CH-1207 Genève (CH); OHLEYER, Eric, F-74350 Cruseilles (FR)
(74) Mandataire: Salvaterra-Garcia, Maria de Lurdes
(86) Numéro de dépôt international: CH9200202
(87) Numéro de publication internationale: WO9308293

(56) Documents cités:
- US-A- 5 071 762
- CHEMICAL ABSTRACTS, vol. 79, no. 19, 12 Novembre 1973, Columbus, Ohio, US; abstract no. 113982w, FUJISHIMA, TETSURO '2-Furoic acid.' page 235 ;
- CHEMICAL ABSTRACTS, vol. 113, no. 5, 30 Juillet 1990, Columbus, Ohio, US; abstract no. 37571q, NEMIROVSKII,V.D. ET AL. 'Conversion of aromatic and furan compounds during cultivation of microorganisms' page 329 ;
- CHEMICAL ABSTRACTS, vol. 106, no. 3, 5 Janvier 1987, Columbus, Ohio, US; abstract no. 17018s, FURAKAWA, TOSHIRO ET AL. 'Microbial oxidation of 1,3- bis(2-hydroxyethoxy)-benzene' page 476 ;
- CHEMICAL ABSTRACTS, vol. 101, no. 19, 5 Novembre 1984, Columbus, Ohio, US; abstract no. 169112c, MITSUBISHI 'Production of monocarboxylic acids from Candida culture.' page 539 ;
- CHEMICAL ABSTRACTS, vol. 111, no. 17, 23 Octobre 1989, Columbus, Ohio, US; abstract no. 152189s, KATO,NOBUO 'Enzymic oxidation of alcohols to acids.' page 580 ;
- CHEMICAL ABSTRACTS, vol. 77, no. 21, 20 Novembre 1972, Columbus, Ohio, US; abstract no. 138355r, ARIMA, KEI ET AL. 'Fermentative production of beta- methylthiopropionic acid.' page 313 ;
- CHEMICAL ABSTRACTS, vol. 89, no. 17, 23 Octobre 1978, Columbus, Ohio, US; abstract no. 144950v, GUVEN, KASIM CEMAL 'Microbiological conversion of geraniol and citral.' page 457 ;

## Description

### Domaine technique

La présente invention a trait au domaine de la synthèse bioorganique. Plus particulièrement, elle concerne un procédé pour la préparation d'acides carboxyliques à partir d'alcools ou aldéhydes correspondants, procédé qui a recours à l'action de certains microorganismes du genre Saccharomyces, Hansenula, Pichia, Candida ou Kluyveromyces.

### Technique antérieure

Parmi les composés dont l'emploi est particulièrement important dans la pratique de l'aromatisation d'aliments ou boissons en général, tout comme dans la parfumerie, figurent bon nombre d'acides carboxyliques ainsi que leurs dérivés esters. Employés en l'état ou servant à titre de matière première pour l'obtention de dérivés de nature variée, ces acides ou esters constituent une classe de composés pour lesquels l'industrie a déployé des efforts constants en vue de leur synthèse. Quoique la production par voie microbienne de certains acides organiques soit connue depuis fort longtemps - il suffit en effet de penser à la formation d'acide acétique lors de la production bactérienne de vinaigre -, à ce jour, nous ne disposons pas de méthodes de biotransformation simples et efficaces pour la fabrication d'acides carboxyliques, méthodes qui soient d'emploi général. Or, c'est précisément et surtout dans certains domaines particuliers comme la pharmacie ou l'alimentation que de telles méthodes microbiologiques se justifient.

Dans la pratique industrielle, nombreuses sont les méthodes qui ont recours à des cultures de Saccharomyces, compte tenu de leur disponibilité, leur bas prix et leur statut en tant que microorganismes agréés dans la préparation d'ingrédients alimentaires. L'emploi donc d'une telle famille de microorganismes est bien documenté dans la littérature.

Il en va de même pour d'autres familles de microorganismes tels ceux du genre Hansenula, Pichia, Candida ou Kluyveromyces.

D'autre part, des réactions de dismutation oxydo-réductives sur des substrats variés ont été décrites dans l'art antérieur. Il est connu par exemple que, sous l'action de la lactate déhydrogénase, l'acide glyoxylique se transforme en acide oxalique et en acide glycolique [Ital. J. Biochem., 20 (1971) 129]. Connue est également la dismutation d'aldéhyde formique en acide formique et méthanol, dismutation induite par l'alcool déhydrogénase [Arch. Biochem. and Biophysics, 141 (1970) 632] ainsi que par des microorganismes de l'espèce Pseudomonas putida [Agr. Biol. Chem., 48 (1984) 2017].

Malgré cela, aucune de ces références ne mentionne ou ne suggère l'utilisation de Saccharomyces, ou des microorganismes du genre Hansenula, Pichia, Candida ou Kluveromyces, dans des procédés d'obtention d'acides carboxyliques par oxydation complète des alcools ou aldéhydes correspondants.

D'autre part le brevet japonais 73 03394, cité dans le Chem. Abst. 1973, 79, page 235, abstract no. 113982W, décrit un procédé de production d'acide α-furoïque par culture de levures du genre *Candida, Torulopsis, Pichia* ou *Rhodotorula* en présence de furfural. L'exemple cité a trait à l'utilisation de *Torulopsis bacillaris* dans un milieu nutritif, contenant du glucose et une concentration en furfural de 2 g par litre de milieu de bioconversion. Le rendement en acide furoïque est de 1,3 g/l de milieu et il est spécifié que le substrat est ajouté pendant la phase de croissance, c'est-à-dire en milieu nutritif. Ce procédé produit des rendements beaucoup trop faibles en acide pour être rentable à l'échelle industrielle et ceci malgré le fait que le milieu de réaction est très dilué en substrat, à savoir le furfural.

### Exposé de l'invention

La présente invention a donc pour objet un procédé biocatalytique de production d'acides carboxyliques par oxydation complète des alcools ou aldéhydes correspondants à l'aide de microorganismes du genre Saccharomyces, Hansenula, Pichia, Candida ou Kluyveromyces.

Plus particulièrement, l'invention a trait à un procédé biocatalytique de production d'acides carboxyliques par oxydation des alcools ou aldéhydes correspondants de formule

RCH₂OH, respectivement RCHO

dans laquelle R sert à désigner un radical aralkyle, alkyle ou alkényle, linéaire ou ramifié, substitué ou non par un groupe thio-alkyle, ou un reste furyle ou furylalkyle, à l'aide d'une levure choisie parmi les microorganismes du genre *Saccharomyces, Hansenula, Pichia, Candida* ou *Kluyveromyces,* caractérisé en ce qu'il comprend :
a) la mise en contact d'un substrat constitué par l'alcool ou l'aldéhyde approprié avec une biomasse desdits microorganismes obtenue par culture aérobique et de façon que les microorganismes ne comportent aucune réserve endogène de carbohydrates, cette mise en contact ayant lieu dans des conditions aérobiques et dans un milieu aqueux non nutritif ayant un pH d'environ 7,0-9,0, pendant une période suffisante pour obtenir l'oxydation quantitative complète dudit alcool ou aldéhyde en l'acide carboxylique correspondant ;
   et
b) l'extraction de l'acide carboxylique ainsi formé du milieu de bioconversion à l'aide d'une méthode conventionnelle.

Selon un mode d'exécution préférentiel de ce procédé, on suspend dans l'eau les microorganismes sous conditions aérobiques à une température d'environ 15 à 35°C, le pH de la suspension étant d'environ 4,5-6,0, pendant une période suffisante pour consommer leur réserve endogène éventuelle de carbohydrates avant la mise en contact avec le substrat, laquelle a lieu par addition subséquente dudit substrat à la suspension résultante.

A titre préférentiel, on utilise comme microorganismes des Saccharomyces cerevisiae (levure boulangère), ce qui rend le procédé particulièrement économique. Parmi les microorganismes préférentiels figurent également ceux du genre Hansenula, en particulier l'Hansenula polymorpha, par exemple de la souche ATCC 46059. L'efficacité du procédé est par ailleurs tout à fait exceptionnelle dans un certain nombre d'exemples. Nous avons constaté en effet que lors de l'oxydation du furfurol en acide furoïque, la biotransformation permettait d'obtenir des concentrations proches de 100 g/l d'acide dans le bouillon de culture. Il en est de même pour l'oxydation du 3-méthylthio-propanol où des concentrations de 45 g/l d'acide correspondant ont été obtenues.

Comme indiqué plus haut, le symbole R peut représenter un radical aralkyle, alkyle ou alkényle, linéaire ou ramifié. C'est ainsi qu'il peut désigner par exemple un radical benzyle, méthyle, éthyle, propyle, isopropyle, butyle, isobutyle, tert-butyle, pentyle, hexyle ou encore 2,6-diméthyl-hept-5-ène-1-yle et 2,6-diméthyl-hepta-1,5-diène-1-yle. Le radical alkyle peut être substitué par un groupe thio-alkyle. Tel est le cas par exemple du radical 2-thiométhyl-éthyle.

Parmi les alcools pouvant ainsi être oxydés en leurs acides correspondants selon le procédé de l'invention, il convient de mentionner à titre d'exemple le butanol, le 2-méthyl-butanol, le 3-méthyl-butanol, l'isobutanol, le n-pentanol, le n-heptanol, le 3-méthylthio-propanol, le citronellol, le géraniol et le furfurol, le phényléthanol, l'alcool vanillique ou cinnamique.

Parmi les aldéhydes, nous pouvons mentionner le n-heptanal, le citral et le furfural.

Quoique l'addition du substrat, constitué par l'alcool ou l'aldéhyde choisi, à la suspension du microorganisme se fait de préférence à un pH compris entre 7,0 et 9,0, des valeurs de pH plus élevées peuvent également être employées. Nous avons en effet remarqué que la culture était particulièrement stable à pH 9,0, ou plus, ainsi que montré par son pouvoir d'oxydation sur le substrat, même après avoir été maintenue plusieurs jours à ce pH.

Le procédé peut être mis en oeuvre en utilisant les techniques et l'appareillage conventionnels d'emploi courant dans les réactions biocatalytiques. Le biocatalyseur peut être constitué par des microorganismes soit à l'état libre, soit sous forme immobilisée.

Comme il a été indiqué plus haut, selon un mode d'exécution particulier, la première phase du procédé consiste à aérer sous agitation une suspension dans l'eau du microorganisme, et ce de façon à favoriser la consommation de leur réserve endogène éventuelle de carbohydrates. Le pH d'une telle suspension se situe dans la région d'environ 4,5-6,0. Une fois terminée cette première phase, la valeur du pH est portée à environ 7,0-9,0 par l'addition graduelle d'une solution alcaline, puis la valeur atteinte est maintenue constante au cours de la phase d'addition du substrat par un système approprié de titration.

Le procédé peut être effectué par des opérations répétitives (en "batch") ou en continu. Dans ce dernier cas, l'acide formé est récolté au fur et à mesure de sa formation et séparé de la biomasse, par exemple par ultrafiltration et chromatographie sur colonne remplie d'une résine échangeuse ionique. Le système en continu permet d'éviter l'accumulation de l'acide formé, accumulation qui, dans certains cas selon la nature de l'acide, aurait comme conséquence d'inhiber la réaction.

La présente invention a également pour objet un mode d'exécution particulier du procédé destiné à la production microbiologique de l'acide 3-méthylthio-propanoïque, lequel est caractérisé en ce que le 3-méthylthio-propanol est engendré dans le milieu de la bioconversion par culture en phase de croissance, sous conditions aérobiques et à un pH d'environ 4,0-5,0, de microorganismes du genre *Saccharomyces* en présence d'un substrat nutritif contenant un carbohydrate et, comme source unique d'azote, de la L-méthionine, et en ce qu'on interrompt par la suite l'alimentation en carbohydrate et ajuste le pH à une valeur proche de 7,0-9,0 et qu'on maintient la suspension résultante sous agitation dans des conditions aérobiques pendant un temps suffisant pour transformer le 3-méthylthio-propanol formé en l'acide correspondant désiré.

Ainsi, dans la première phase, on engendre le 3-méthylthio-propanol dans le milieu par culture, en phase de croissance, des microorganismes du genre Saccharomyces en présence d'un substrat nutritif contenant un carbohydrate, de préférence du glucose, et de la L-méthionine, comme source unique d'azote.

Dans la deuxième phase, on arrête l'addition du glucose et ajuste la valeur du pH de la suspension obtenue à environ 9,0 de préférence, ce qui dans un premier temps, entraîne l'oxydation de l'éthanol présent en acide acétique, puis, une fois cette étape terminée, l'oxydation du 3-méthylthio-propanol en l'acide correspondant, 3 -méthylthio-propanoïque.

L'acide ainsi formé peut être séparé de la biomasse au moyen des techniques usuelles telles que mentionnées plus haut.

Dans ce cas également, la biotransformation de la L-méthionine en acide 3-méthylthio-propanoïque peut être effectuée à l'aide d'un appareillage conventionnel. Comme substrat nutritif, on emploie, en tant qu'adjuvants du carbohydrate et de la L-méthionine, des sels tels le phosphate de potassium, le sulphate de magnésium, le chlorure de calcium, le sulphate de fer^{II}, le sulphate de zinc, le sulphate de manganèse et le sulphate de cuivre^{II}. A ce milieu nutritif, on peut également ajouter de la biotine.

A titre de biocatalyseur, on peut employer des microorganismes du genre Saccharomyces, en particulier des Saccharomyces cerevisiae ou levure boulangère telle qu'offerte sur le marché. D'autres microorganismes, notamment ceux du genre Hansenula ou Pichia, se sont montrés tout aussi appropriés.

Des concentrations variables de tels microorganismes peuvent être employées. Il en va de même pour les proportions de substrat nutritif. Ainsi, les quantités de levure peuvent varier entre environ 5 et 50% en poids dans le milieu de culture. Quant à la solution nutritive, elle peut être convenablement ajoutée au milieu de culture par un système d'introduction graduelle. Dans la pratique, on procède en mettant en suspension la levure boulangère dans une solution aqueuse contenant un mélange initial de substrat nutritif. Après ajustage du pH à environ 4,5, la suspension résultante est maintenue sous agitation dans des conditions appropriées d'aérobie, ce qui est assuré par l'introduction controlée d'un courant d'air. Le régime désiré de croissance est ensuite atteint par l'adjonction graduelle d'une solution contenant le carbohydrate et la L-méthionine. Une telle solution contient de préférence environ 50% de glucose et environ 3-5% de L-méthionine.

Enfin, après bioconversion complète de la méthionine en 3-méthylthio-propanol, l'addition de la solution nutritive est interrompue et le pH est ajusté à environ 9,0. Sous ces conditions, la fermentation continue avec la transformation du 3-méthylthio-propanol en acide 3-méthylthio-propanoïque.

L'invention est illustrée de manière plus détaillée par les exemples suivants dans lesquels les températures sont indiquées en degrés centigrades et les abréviations ont le sens usuel dans l'art.

### Exemple 1

### Méthode générale d'expérimentation

Afin d'établir les conditions les meilleures d'expérimentation, on a effectué ainsi la préparation d'acides carboxyliques.

Une suspension de 500 ml de levure boulangère à 20% dans l'eau a été maintenue pendant une nuit à 30° sous agitation dans un flacon Erlenmeyer de 1 l. Ensuite, 50 ml de cette suspension ont été placés dans un flacon Erlenmeyer de 125 ml et 2 g de carbonate de calcium y ont été ajoutés de façon a ajuster le pH à environ 7,0; puis, 50 mg du substrat choisi, alcool ou aldéhyde, ont été additionnés au mélange résultant et le tout a été agité jusqu'à réaction complète. Le déroulement de la réaction a été suivi par le prélèvement régulier d'aliquotes de 1 ml chacune.
Celles-ci ont été centrifugées de façon à obtenir un liquide clair. Des échantillons de ce liquide (500 µl) ont été acidifiés avec 50 µl d'HCl 5N et extraits avec 500 µl d'acétate d'éthyle dans un tube Eppendorf. La phase organique a été analysée par chromatographie gazeuse à l'aide d'une colonne mod. SPB1 [origine : Supelco].
En procédant comme décrit ci-dessus, on a préparé les acides carboxyliques désignés dans le tableau suivant :

| Produit de départ | Acide carboxylique |
|---|---|
| Alcools | |
| Isobutanol | ac. isobutyrique |
| 2-Méthyl-butanol | ac. 2-méthyl-butyrique |
| 3-Méthyl-butanol | ac. 3-méthyl-butyrique |
| n-Pentanol | ac. pentanoïque |
| n-Heptanol | ac. heptanoïque |
| 3-Méthylthio-propanol | ac. 3-méthylthio-propanoïque |
| Citronellol | ac. 3,7-diméthyl-oct-6-ène-1-oïque |
| Géraniol | ac. géranique |
| Furfurol | ac. furoïque |
| Phényléthanol | ac. phénylacétique |
| Alcool vanillique | ac. vanillique |
| Alcool cinnamique | ac. cinnamique |
| | |

| Aldéhydes | |
|---|---|
| n-Heptanal | n-heptanoïque |
| Citral | ac. géranique |
| Furfural | ac. furoïque |

La nature des produits obtenus a été déterminée par comparaison avec des échantillons purs commerciaux.

Parmi les substrats testés comme indiqué plus haut, on a choisi les composés suivants pour bioconversion dans un fermenteur : isobutanol, 3-méthylthio-propanol, heptanal, furfural et furfurol. La méthode suivie est décrite ci-après à titre d'exemple pour la production d'acide furoïque.

800 ml d'une suspension de levure boulangère à 20% (poids/volume) dans l'eau ont été maintenus à 30° sous agitation pendant une nuit (1300 rpm) dans un réacteur de 1 l 5 cols équipé d'une turbine (Medimex), d'un titrateur pH, d'un tube d'alimentation d'air (2,5 v/v/m) et d'un réfrigérant. Le pH a été ensuite augmenté de 3,5 à 9,0 par l'adjonction de 4,85 mM de soude dans un intervalle d'environ 10 min. Le pH a été maintenu à la valeur indiquée par l'addition graduelle de soude, et ce pendant plusieurs heures, puis 70 ml (0,81 M) de furfurol ont été introduits dans le réacteur par aliquotes successives de 10 ml chacune. L'addition a lieu à 0, 3, 8, 23, 27, 31 et 47 heures. Après 62 h le mélange de réaction (850 ml) a été centrifugé pour fournir 705 ml d'un liquide clair brun qui a été acidifié avec 85 ml d'HCl conc., ce qui a provoqué la précipitation de l'acide désiré. Par filtration et séchage sous vide, on a obtenu 52,2 g d'un solide amorphe légèrement brun. La phase aqueuse a été extraite à l'éther (2x700 ml), puis la partie éthérée a été séchée sur du MgSO₄ et évaporée pour donner une fraction supplémentaire de 24,3 g d'acide furoïque.
L'acide isolé était identique à un échantillon analytique obtenu à partir d'un produit commercial.

### Exemple 2

On a préparé un mélange initial de fermentation par mélange des ingrédients suivants :

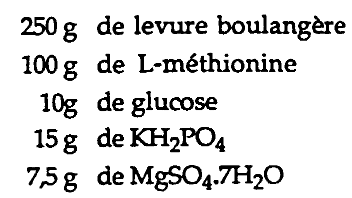

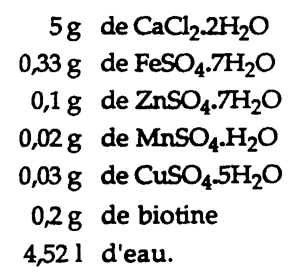

Ce mélange a été placé dans un fermenteur à 30°. Le pH a été ajusté à 4,5 et maintenu à cette valeur par l'addition d'une solution de soude à 30%, tandis qu'un courant d'air (10 l/min) a été introduit dans la suspension sous agitation (env. 300-500 rpm).
Une solution nutritive de glucose et L-méthionine a été ensuite ajoutée graduellement au mélange dans les intervalles et quantités suivants:

| Temps | Solution | Quantité [l] | Vitesse d'addition [ml/h] |
|---|---|---|---|
| 0-15 h | 50% glucose | 1,0 | 66,7 |
| 16-60 h | 50% glucose + 3,33% L-méthionine | 3,0 | 66,7 |
| 61-95 h | 50% glucose | 2,34 | 66,7 |

L'adjonction de solution nutritive a été arrêtée et le pH a été porté à environ 9,0, puis la fermentation a été poursuivie pendant 88 h.
Le mélange ainsi obtenu (9,75 kg) a été centrifugé pour fournir 8,64 l d'un liquide clair brun dont une aliquote de 500 ml a été extraite avec de l'acétate d'éthyle (2x500 ml), puis la phase aqueuse a été acidifiée à pH 1 avec de l'HCl conc. et extraite à nouveau avec deux fractions (500 ml) d'acétate d'éthyle.
Les extraits organiques ont été lavés avec une solution saline, séchés sur du MgSO₄ anhydre et filtrés. Par évaporation du solvant, on a obtenu comme résidu 9,1 g d'une huile foncée qui, par distillation dans un appareil à boules (0,1 mbar; 125°) ont fourni 5,64 g d'acide 3-méthylthio-propanoïque.
Les caractères analytiques de l'acide obtenu étaient en tous points identiques à ceux d'un échantillon analytique préparé à partir d'un produit commercial.

### Exemple 3

### Oxydation d'alcool isoamylique à l'aide d'Hansenula polymorpha

### 1. Fermentation

Une masse biologique d'Hansenula polymorpha ATCC46059 a été obtenue par fermentation aérobique en utilisant le milieu de culture suivant [g/l]:

| | |
|---|---|
| glucose | 25 |
| extrait de levure | 10 |
| peptone | 10 |
| K H₂PO₄ | 5 |
| NaCl | 2 |

15l de ce mélange ont été placés dans un fermenteur équipé d'une entrée d'aération (5 v/v/m) et le pH a été ajusté à 7,0 tandis que la température était portée à 37°. La masse biologique humide a été récoltée par centrifugation, puis les cellules ont été lavées une fois avec de l'eau distillée et recentrifugées. Elles sont ainsi prêtes pour l'étape suivante de bioconversion.

### 2. Bioconversion

La bioconversion avec les cellules en survie est réalisée à température ambiante, dans l'eau avec 10% de biomasse humide et 0,01% d'alcool isoamylique.
Le progrès de la bioconversion a été suivi par analyse chromatographique d'échantillons du milieu réactionnel après extraction à l'éther.
La réaction est poursuivie pendant 6 jours. L'acide isovalérianique est ainsi obtenu avec un taux de conversion de 90%.

## Revendications

1. Procédé biocatalytique de production d'acides carboxyliques par oxydation des alcools ou aldéhydes correspondants de formule
RCH₂OH, respectivement RCHO
dans laquelle R sert à désigner un radical aralkyle, alkyle ou alkényle, linéaire ou ramifié, substitué ou non par un groupe thio-alkyle, ou un reste furyle ou furylalkyle, à l'aide d'une levure choisie parmi les microorganismes du genre *Saccharomyces, Hansenula, Pichia, Candida* ou *Kluyveromyces,* caractérisé en ce qu'il comprend :
a) la mise en contact d'un substrat constitué par l'alcool ou l'aldéhyde approprié avec une biomasse desdits microorganismes obtenue par culture aérobique et de façon à ce que les microorganismes ne comportent aucune réserve endogène de carbohydrates, cette mise en contact ayant lieu dans des conditions aérobiques et dans un milieu aqueux non nutritif ayant un pH d'environ 7,0-9,0, pendant une période suffisante pour obtenir l'oxydation quantitative complète dudit alcool ou aldéhyde en l'acide carboxylique correspondant;
et
b) l'extraction de l'acide carboxylique ainsi formé du milieu de bioconversion à l'aide d'une méthode conventionnelle.

2. Procédé selon la revendication 1, caractérisé en ce qu'on suspend dans l'eau les microorganismes sous conditions aérobiques à une température d'environ 15 à 35°C, le pH de la suspension étant d'environ 4,5-6,0, pendant une période suffisante pour consommer leur réserve endogène éventuelle de carbohydrates avant la mise en contact avec le substrat, laquelle a lieu par addition subséquente dudit substrat à la suspension résultante.

3. Procédé selon la revendication 1 ou 2, caractérisé en ce que les microorganismes appartiennent à l'une des espèces *Saccharomyces cerevisiae* (levure boulangère) ou *Hansenula polymorpha.*

4. Procédé selon l'une quelconque des revendications précédentes, caractérisé en ce que l'acide formé est extrait du milieu de bioconversion par un système en continu à l'aide d'ultrafiltration combinée à de la chromatographie par échange ionique.

5. Procédé selon l'une quelconque des revendications précédentes, caractérisé en ce qu'on soumet à oxydation quantitative complète le 3-méthylthio-propanol ou le 3-méthylthio-propanal et qu'on obtient l'acide 3-méthylthio-propanoïque.

6. Procédé selon la revendication 5, caractérisé en ce que le 3-méthylthio-propanol est engendré dans le milieu de la bioconversion par culture en phase de croissance, sous conditions aérobiques et à un pH d'environ 4,0-5,0, de microorganismes du genre *Saccharomyces* en présence d'un substrat nutritif contenant un carbohydrate et, comme source unique d'azote, de la L-méthionine, et en ce qu'on interrompt par la suite l'alimentation en carbohydrate et ajuste le pH à une valeur proche de 7,0-9,0 et qu'on maintient la suspension résultante sous agitation dans des conditions aérobiques pendant un temps suffisant pour transformer le 3-méthylthio-propanol formé en l'acide correspondant désiré.

7. Procédé selon l'une quelconque des revendications 1 à 4, caractérisé en ce qu'on soumet à oxydation quantitative complète le furfurol ou le furfural et qu'on obtient l'acide furoïque.

8. Procédé selon l'une quelconque des revendications 1 à 4, caractérisé en ce qu'on soumet à oxydation quantitative complète le géraniol et qu'on obtient l'acide géranique.

9. Procédé selon l'une quelconque des revendications 1 à 4, caractérisé en ce qu'on soumet à oxydation quantitative complète le butanol, le 2-méthyl-butanol ou le 3-méthyl-butanol et qu'on obtient l'acide carboxylique correspondant.

10. Procédé selon l'une quelconque des revendications précédentes, caractérisé en ce que le pH du milieu aqueux est ajusté à une valeur d'environ 9,0 avant la mise en contact du substrat avec les microorganismes et maintenu à cette valeur pendant toute la période de contact nécessaire pour l'oxydation quantitative complète du substrat.

## Patentansprüche

1. Biokatalytisches Verfahren zur Herstellung von Carbonsäuren durch Oxidation von entsprechenden Alkoholen oder Aldehyden der Formel
RCH₂OH beziehungsweise RCHO
worin R einen geradekettigen oder verzweigten, durch eine Thioalkylgruppe substituierten oder unsubstituierten Aralkyl-, Alkyl- oder Alkenylrest, oder einen Furyl- oder Furylalkyl-Rest bedeutet, mit Hilfe einer Hefe, ausgewählt unter den Mikroorganismen der Gattung *Saccharomyces, Hansenula, Pichia, Candida oder Kluyveromyces,* dadurch gekennzeichnet, dass dieses umfaßt:
a) das Inkontaktbringen eines Substrats, welches aus dem entsprechenden Alkohol oder Aldehyd besteht, mit einer Biomasse dieser Mikroorganismen, welche durch aerobe Kultur und in der Weise erhalten wurde, dass die Mikroorganismen keine endogene Reserve von Kohlehydraten enthalten, dieses Inkontaktbringen findet unter aeroben Bedingungen und in einem wässrigen, nicht nährendem Medium mit einem pH Wert von ca. 7,0-9,0 während eines genügend langen Zeitabschnittes statt, um eine mengenmässig vollständige Oxidation dieses Alkohols oder Aldehyds zur entsprechenden Carbonsäure zu erhalten; und
b) die Extraktion der so gebildeten Carbonsäure aus dem Biokonversionsmedium mit einer üblichen Methode.

2. Verfahren gemäss Patentanspruch 1, dadurch gekennzeichnet, dass man die Mikroorganismen unter aeroben Bedingungen bei einer Temperatur von ca. 15 bis 35 C in Wasser suspendiert, wobei der pH der Suspension ca. 4,5-6,0 beträgt, was während eines genügend langen Zeitabschnittes erfolgt, um die eventuelle endogene Reserve der Kohlehydrate vor dem Inkontaktbringen mit dem Substrat zu verbrauchen, welches durch nachfolgende Zugabe dieses Substrats zur entstandenen Suspension vorgenommen wird.

3. Verfahren gemäss den Patentansprüchen 1 oder 2, dadurch gekennzeichnet, dass die Mikroorganismen zu einer der Gattungen *Saccharomyces cerevisae* (Backhefe) oder *Hansenula polymorpha* gehören.

4. Verfahren gemäss einem der vorhergehenden Patentansprüche, dadurch gekennzeichnet, dass die gebildete Säure aus dem Biokonversionsmedium durch ein kontinuierliches System mit Hilfe von Ultrafiltration, kombiniert mit Ionenaustauschchromatographie, extrahiert wird.

5. Verfahren gemäss einem der vorhergehenden Patentansprüche, dadurch gekennzeichnet, dass man 3-Methylthiopropanol oder 3-Methylthiopropanal der mengenmässig vollständigen Oxidation unterwirft und 3-Methylthiopropansäure erhält.

6. Verfahren gemäss Patentanspruch 5, dadurch gekennzeichnet, dass 3-Methylthiopropanol im Biokonversionsmedium durch die im Wachstumszustand befindliche Kultur von Mikroorganismen der Gattung *Saccharomyces* unter aeroben Bedingungen und einem pH von ca. 4,0-4,5 in Anwesenheit eines Nährsubstrats, welches ein Kohlehydrat und als alleinige Stickstoffquelle L-Methionin enthält, gebildet wird und man danach die Zufuhr des Kohlehydrats unterbricht und den pH auf einen Wert nahe zu 7,0-9,0 einstellt und die gebildete Suspension unter aeroben Bedingungen während eines genügend langen Zeitabschnittes rührt, um den gebildeten 3-Methylthiopropanol in die gewünschte entsprechende Säure zu überführen.

7. Verfahren gemäss einem der Patentansprüche 1 bis 4, dadurch gekennzeichnet, dass man Furfurol oder Furfural einer mengenmässig vollständigen Oxidation unterwirft und Furancarbonsäure erhält.

8. Verfahren gemäss einem der Patentansprüche 1 bis 4, dadurch gekennzeichnet, dass man Geraniol einer mengenmässig vollständigen Oxidation unterwirft und die Geraniumsäure erhält.

9. Verfahren gemäss einem der Patentansprüche 1 bis 4, dadurch gekennzeichnet, dass man Butanol, 2-Methylbutanol oder 3-Methylbutanol einer mengenmässig vollständigen Oxidation unterwirft und man die entsprechende Carbonsäure erhält.

10. Verfahren gemäss einem der vorhergehenden Patentansprüche, dadurch gekennzeichnet, dass der pH-Wert des wässrigen Mediums auf einen Wert von ca. 9,0 vor dem Inkontaktbringen des Substrats mit den Mikroorganismen eingestellt und bei diesem Wert während der gesamten für die mengenmässig vollständige Oxidation des Substrats notwendigen Kontaktzeit gehalten wird.

## Claims

1. Biocatalytic process for producing carboxylic acids via oxidation of the corresponding alcohols or aldehydes of formula
RCH₂OH, respectively RCHO
wherein R designates an aralkyl, alkyl or alkenyl radical, linear or branched, substituted or not by a thioalkyl group, or a furyl or furylalkyl radical, by means of a yeast selected amongst the microorganisms of the *Saccharomyces, Hansenula, Pichia, Candida* or *Kluyveromyces* genus, characterized in that it comprises :
a) contacting a substrate consisting of the appropriate alcohol or aldehyde with a biomass of said microorganisms obtained by aerobic culture and in such a way that the microorganisms possess no endogenous carbohydrate supply, said contact taking place under aerobic conditions and in a non-nutrient aqueous medium having a pH of about 7.0-9.0, for an amount of time sufficient to obtain the complete quantitative oxidation of said alcohol or aldehyde into the corresponding carboxylic acid ;
and
b) extracting the thus formed carboxylic acid from the bioconversion medium by means of a conventional method.

2. Process according to claim 1, characterized in that the microorganisms are suspended in water under aerobic conditions at a temperature of about 15 to 35°C, the pH of the suspension being of about 4.5-6.0, for an amount of time sufficient to consume their potential endogenous carbohydrate supply before contacting with the substrate, which contact occurs via subsequent addition of said substrate to the resulting suspension.

3. Process according to claim 1 or 2, characterized in that the microorganisms belong to one of the *Saccharomyces cerevisiae* (baker's yeast) or *Hansenula polymorpha* species.

4. Process according to any one of the preceding claims, characterized in that the formed acid is extracted from the bioconversion medium via a continuous system by means of ultrafiltration combined with ionic exchange chromatography.

5. Process according to any one of the preceding claims, characterized in that 3-methylthio-propanol or 3-methylthio-propanal is subjected to complete quantitative oxidation and in that 3-methylthio-propionic acid is obtained.

6. Process according to claim 5, characterized in that 3-methylthio-propanol is generated in the bioconversion medium by growth-phase culture, under aerobic conditions and at a pH of about 4.0-5.0, of microorganisms of the *Saccharomyces* genus in the presence of a nutrient substrate containing a carbohydrate and, as sole nitrogen source, L-methionine, and in that the supply of carbohydrate is subsequently interrupted and the pH is adjusted to a value close to 7.0-9.0 and the resulting suspension is kept under stirring under aerobic conditions for an amount of time sufficient to convert the formed 3-methylthio-propanol into the desired corresponding acid.

7. Process according to any one of the claims 1 to 4, characterized in that furfurol or furfural is subjected to complete quantitative oxidation and in that one obtains furoic acid.

8. Process according to any one of the claims 1 to 4, characterized in that geraniol is subjected to complete quantitative oxidation and in that one obtains geranic acid.

9. Process according to any one of the claims 1 to 4, characterized in that butanol, 2-methyl-butanol or 3-methyl-butanol is subjected to complete quantitative oxidation and in that one obtains the corresponding carboxylic acid.

10. Process according to any one of the preceding claims, characterized in that the pH of the aqueous medium is adjusted to a value of about 9.0 before contacting the substrate with the microorganisms and maintained at this value during the whole contacting time necessary for complete quantitative oxidation of the substrate.
